# FASCICULE DE BREVET EUROPEEN

(11) **EP 1 152 704 B1**
(45) Date de publication et mention de la délivrance du brevet: **15.09.2004**
(21) Numéro de dépôt: 00906430.4
(22) Date de dépôt: 18.02.2000
(51) Int. Cl.: A61B 17/70

(54) **DISPOSITIF DE DISTRACTION/CONTRACTION POUR SYSTEME D'OSTEOSYNTHESE RACHIDIENNE**
DISTRAKTIONS/VERKÜRZUNGSVORRICHTUNG FÜR EIN SYSTEM ZUR WIRBELSÄULENOSTEOSYNTHESE
DISTRACTION/CONTRACTION DEVICE FOR BACKBONE OSTEOSYNTHESIS

(30) Priorité: 18.02.1999 FR 9901987
(43) Date de publication de la demande: 14.11.2001
(73) Titulaire: Dimso (Distribution Médicale du Sud-Ouest), 33610 Cestas (FR)
(72) Inventeur: CONCHY, Frédéric, F-33650 Saint-Médard d'Eyrans (FR); PASQUET, Denis, F-33000 Bordeaux (FR)
(74) Mandataire: Texier, Christian
(86) Numéro de dépôt international: PCT/FR2000/000414
(87) Numéro de publication internationale: WO 2000/048523

(56) Documents cités:
- WO-A-93/14908
- DE-C- 4 007 306
- US-A- 4 926 849
- DATABASE WPI Section PQ, Week 8650 Derwent Publications Ltd., London, GB; Class P31, AN 1986-330695 XP002120733 & SU 1 223 904 A (MOSC REG CLINIC INS)

## Description

La présente invention concerne une instrumentation pour des systèmes d'ostéosynthèse rachidienne.

Lors de la mise en place d'un système d'ostéosynthèse rachidienne, le chirurgien est amené à modifier la distance séparant les deux vertèbres recevant le système d'ostéosynthèse. De plus, lors des mises en place par voie endoscopique, l'utilisation d'une pince classique dite distractrice (utilisée dans le cas où les vertèbres sont à éloigner l'une de l'autre) ou dite contractrice (utilisée dans le cas où les vertèbres sont à rapprocher l'une de l'autre) est problématique voire impossible du fait de son encombrement. D'autre part, les instruments endoscopiques ont des courses trop faibles ou ne permettent pas d'appliquer d'efforts suffisants pour réaliser l'opération de distraction ou de contraction.

Par ailleurs, le document DE 91 12 466 U nous enseigne une instrumentation permettant de positionner deux vis pédiculaires l'une par rapport à l'autre. Cette instrumentation est composée de deux bras s'interfaçant avec les vis pédiculaires et reliés entre eux par deux dispositifs de déplacement présentant chacun deux filetages en sens opposés en prise avec les bras respectifs. L'ensemble de l'instrumentation est très volumineux ce qui la rend inutilisable par voie chirurgicale endoscopique.

Le document WO 93/14 908 divulgue un dispositif conforme au préambule de la revendication 1.

Un but de la présente invention est de fournir un dispositif permettant la distraction et la contraction de deux vertèbres tout en étant utilisable par voie endoscopique et simple d'utilisation.

Selon la présente invention, on prévoit, en vue de réaliser ce but, un dispositif selon la revendication 1.

Ainsi, le dispositif permet de réaliser effectivement une distraction ou une contraction entre deux vertèbres de manière simple tout en conservant le déplacement appliqué par un effet anti-retour sans intervention de l'opérateur.

Des modes de réalisation avantageux sont enoncés aux revendications 2 à 12.

Avantageusement, l'un au moins des connecteurs présente un crochet bifide délimitant un espace apte à recevoir la tige et ses moyens de liaison.

Ainsi le dispositif est démontable en petits éléments, facilitant ainsi son utilisation par voie endoscopique et son assemblage.

Des modes de réalisation avantageux sont enoncés aux revendications 14 à 22.

On prévoit aussi un système d'ostéosynthèse rachidienne comprenant deux organes d'ancrage vertébral ainsi qu'un dispositif de distraction et/ou de contraction selon la présente invention.

Avantageusement, l'un des connecteurs est apte à former une liaison rotule avec l'organe d'ancrage vertébral associé.

Ainsi, le dispositif peut être mis en place sur des organes d'ancrage présentant des défauts d'alignement et de parallélisme.

Avantageusement, la bague est apte à être montée sur l'organe d'ancrage vertébral.

On prévoit aussi une méthode chirurgicale destinée à effectuer une ostéosynthèse rachidienne à l'aide d'un tel système d'ostéosynthèse comprenant un dispositif de distraction et/ou de contraction selon la présente invention : mise en place des organe d'ancrage sur les vertèbres, puis mise en place du dispositif sur les organes d'ancrage et réglage de la distance, puis mise en place du système d'ostéosynthèse, puis retrait du dispositif.

D'autres caractéristiques et avantages de l'invention apparaîtront encore dans la description suivante de trois mode de réalisation préférés donnés à titre d'exemples non limitatifs. Aux dessins annexés :
- la figure 1 est une vue en trois dimensions d'un premier mode de réalisation préféré ;
- la figure 2 est une vue éclatée du premier mode de réalisation ;
- les figures 3a et 3b sont des vues éclatées en trois dimensions sous deux angles différents du premier connecteur ;
- la figure 3c est une vue en coupe axiale du premier connecteur ;
- les figures 4a et 4b sont des vues éclatées en trois dimensions sous deux angles différents du second connecteur ;
- la figure 4c est une vue en coupe axiale du second connecteur ;
- la figure 5 est vue de dessus du premier mode de réalisation mise en place sur une colonne vertébrale ;
- la figure 6 est une vue en trois dimensions d'un deuxième mode de réalisation ;
- la figure 7a est une vue par le dessous en trois dimensions d'un troisième mode de réalisation ; et
- la figure 7b est une vue de dessus en trois dimensions du troisième mode de réalisation.

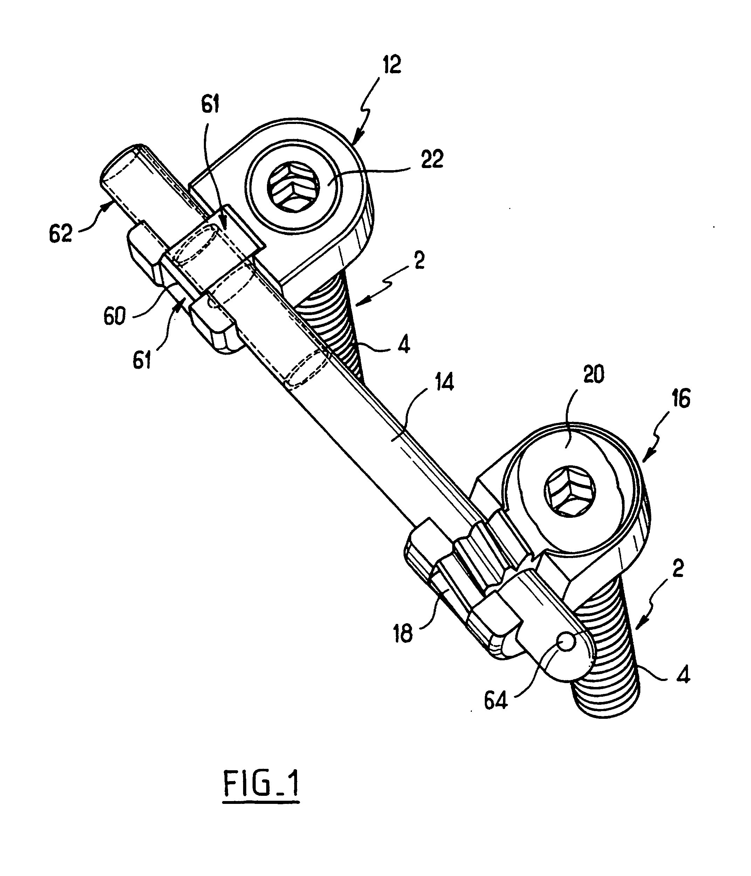

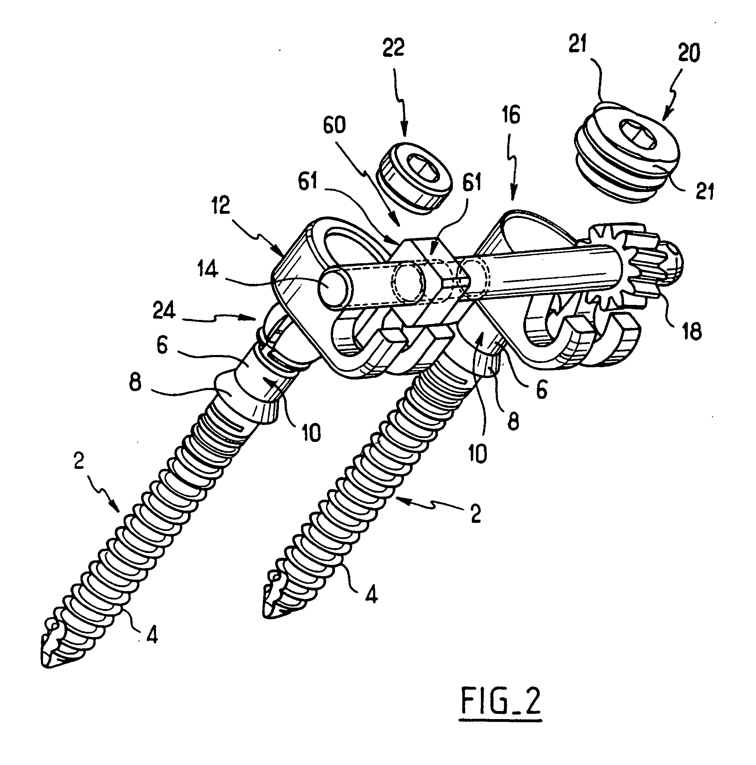

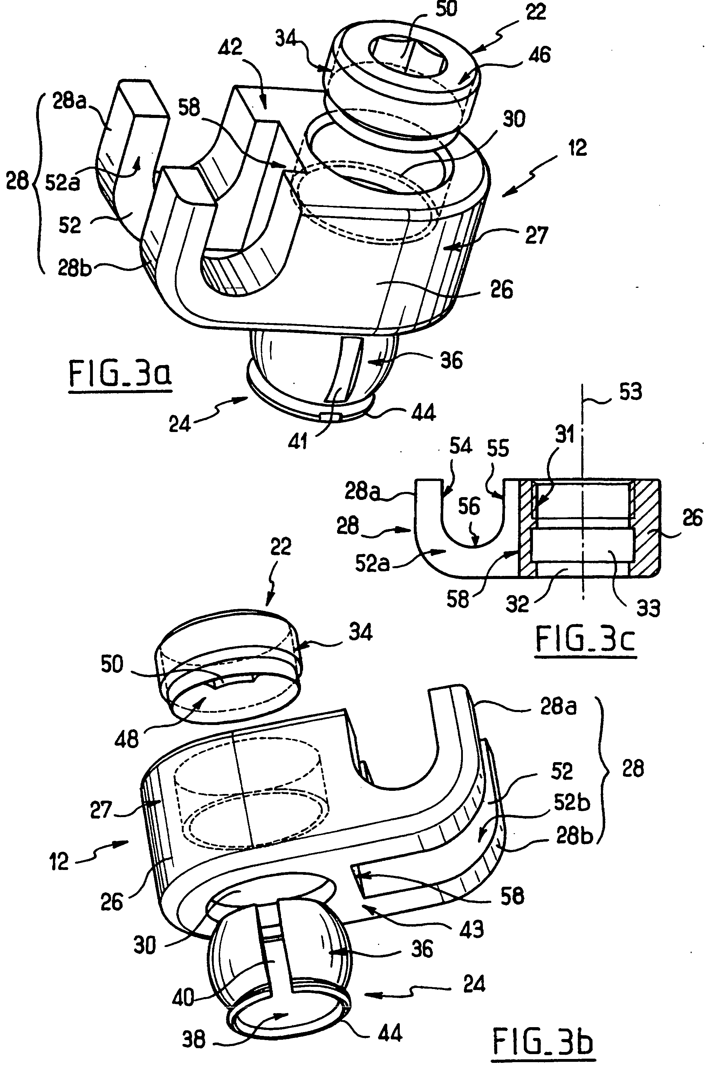

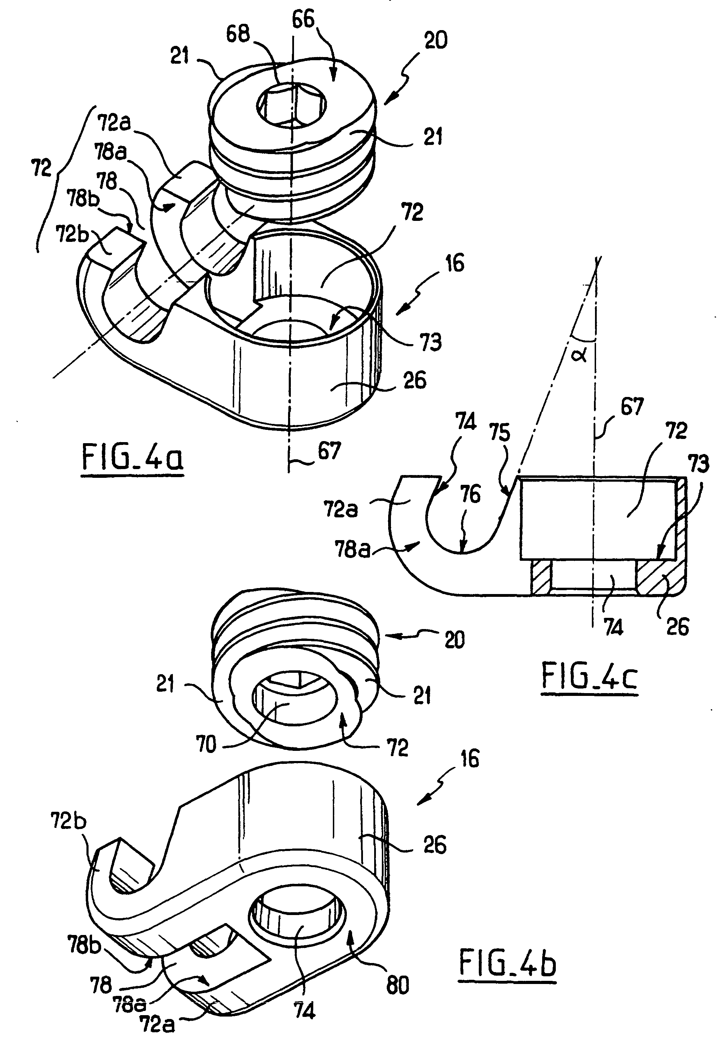

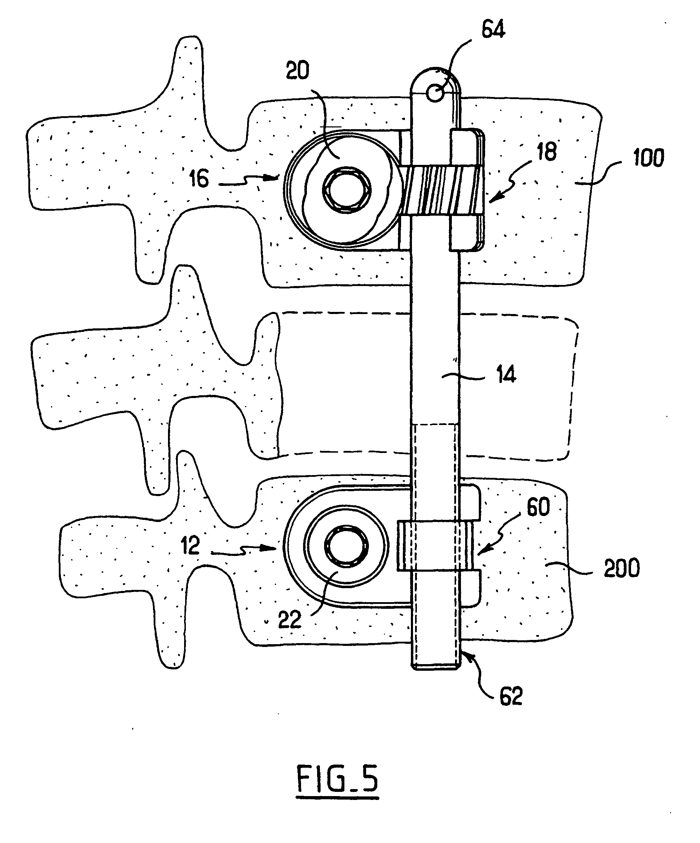

Nous allons décrire un premier mode de réalisation de la présente invention annexé aux figures 1 à 5.

Le système d'ostéosynthèse rachidienne comprend des organes d'ancrage vertébraux ici sous la forme de vis bicorticales 2 comprenant chacune un corps fileté 4 et une tête cylindrique 6 surmontant une collerette 8. La tête cylindrique 6 présente une face externe lisse 10 formant un cylindre d'axe celui de la vis bicorticale 2.

Pour chaque paire de vis bicorticales 2 situées chacune sur un corps vertébral 100, 200, le dispositif comprend un premier connecteur 12, une tige 14, un deuxième connecteur 16 ainsi que des moyens d'entraînement 18 et 20.

Le premier connecteur 12 est constitué d'un corps 26 et d'un crochet de réception 28.

Le corps 26 est de forme générale parallélépipèdique avec une face arrière 27 cylindrique de section hémicirculaire de diamètre égal au coté du parallélépipède. Le corps 26 est traversé de sa face supérieure 42 à sa face inférieure 43 par un orifice 30 cylindrique de section circulaire constitué d'une partie supérieure 31 et d'une partie inférieure 32. La partie supérieure 31 est un trou taraudé dont le filetage est complémentaire du filetage 34 d'un verrou 22 permettant à ce dernier de s'engager dans l'orifice 30. La partie inférieure 32 comporte un trou lisse avec un logement 33 réalisé par un chambrage de section circulaire coaxial avec l'orifice 30 permettant de recevoir et de maintenir prisonnière une bague ou une olivette 24.

L'olivette 24 comporte une face externe 36 lisse de forme sphérique de même rayon que celui du dit chambrage 33 ainsi qu'une face interne 38 lisse, plutôt de forme cylindrique de même diamètre que la face externe lisse 10 de la vis bicorticale 2. L'olivette 24 comprend une fente 40 droite allant de la face externe 36 à la face interne 38 et ce sur toute la longueur de l'olivette 24. Elle comprend en outre une rainure 41 diamétralement opposée à la fente 40 et située sur la face externe 36. Une lèvre 44 de retenue fait saillie sur la face inférieure de l'olivette 24. Cette lèvre 44 évite que l'ouverture de l'orifice délimité par la face interne 38 de l'olivette 24 soit masquée par la rotation de la dite olivette 24 dans son logement 33, ce qui empêcherait le montage du connecteur 12 sur la tête de la vis bicorticale 2.

Le verrou 22 comprend une face supérieure 46 plane et une face inférieure 48 hémisphérique concave sensiblement de même diamètre que la face sphérique 36 de l'olivette 24 et venant coiffer cette dernière lors du montage. Une empreinte 50 hexagonale part de la face supérieure 46 et débouche sur la face inférieure 48 permettant l'adaptation d'un moyen d'entraînement pour la mise en mouvement du verrou 22 dans le but de le visser ou de le dévisser.

Le crochet de réception 28 est bifide. Il est constitué de deux crochets 28a, 28b en forme de « U » séparés par un espace 52. Les branches du « U » sont parallèle entre elles et parallèles à l'axe 53 de l'orifice 30. Le fond 56 des crochets est circulaire pour recevoir la tige 14 lors de l'utilisation du dispositif. L'espace 52 est délimité par une face d'appui 58 parallèle à l'axe 53 de l'orifice 30, d'une part, et, d'autre part, du fond 56 circulaire des crochets 28a, 28b. L'espace 52 est délimité aussi par les faces 52a et 52b formant les cotés des crochets 28a et 28b respectifs. Ainsi, l'espace 52 est configuré de manière à permettre l'insertion d'un plot 60 de section carrée dont l'un des côtés 61 est en contact avec la face d'appui 58, bloquant ainsi le plot 60 en rotation. Les faces 52a et 52b annulent, quant à elles, les translations selon l'axe du crochet de réception.

Dans ce mode préféré de réalisation, la tige 14 présente un filetage 62 qui vient en prise avec le filetage du plot 60, ce dernier faisant alors office d'écrou, se vissant et se dévissant suivant les mouvements de la tige 14. A l'autre extrémité de la tige 14, un orifice 64 de préhension précède un pignon 18 solidaire de la tige 14. L'orifice 64 de préhension permet la manipulation de la tige 14 lors du mode opératoire en voie endoscopique. Les moyens d'entraînement comprennent, en plus du pignon 18, une vis sans fin 20. La vis sans fin 20 comporte au moins un filet 21 d'entraînement.

La pignon 18 est hélicoïdal avec un angle d'hélice correspondant à la pente des filets 21 d'entraînement de la vis sans fin 20 (angle compris entre 5° et 25°).

De la face supérieure 66 de la vis sans fin 60, part une empreinte 68 hexagonale qui permet l'adaptation d'un moyen d'entraînement. Cette empreinte 68 débouche dans une chambre 70 circulaire qui s'étend jusqu'à la face inférieure 72 de la vis sans fin 20. Cette chambre circulaire 70 a un diamètre correspondant au diamètre de la face externe 10 de la tête 6 de la vis bicorticale 2 pour que cette dernière puisse s'y loger sans peine et sans perturber le mouvement en rotation de la vis sans fin 20. L'axe de l'empreinte 68 et l'axe de la chambre circulaire 70 sont confondus avec l'axe 67 de la vis sans fin 20.

Le deuxième connecteur 16 se différencie du premier connecteur 12 en ce qu'il comporte :
- en lieu et place de l'orifice 30 du corps 26 une chambre circulaire 72 pouvant recevoir la vis sans fin 20 : son diamètre est légèrement supérieur à celui de la vis sans fin 20 et sa profondeur est égale à la hauteur de la vis sans fin 20. Du fond 73 de cette chambre de réception 72, un trou 74 coaxial est percé de manière à déboucher sur la face inférieure 80 du second connecteur 16. Le diamètre de ce trou 74 est identique à celui de la chambre circulaire 70 de la vis sans fin 20 dans le but de permettre le passage de la tête 6 de la vis bicorticale 2.
- Un crochet de réception 72 bifide dont les branches 74,75 du « U » forment un angle α avec l'axe 67 de la chambre de réception 72 de manière à ce que l'ouverture des « U » soit vers le corps 26 du connecteur 16. Cet angle α est compris entre 5° et 30°. L'espace 78 entre les deux crochets 72a, 72b est délimité par les faces 78a et 78b formants les côtés des crochets 72a et 72b respectivement. Cet espace 78 pénètre dans la chambre de réception 72 de la vis sans fin 20 d'au moins la profondeur des filets 21 de la dite vis sans fin 20 sans déborder sur le trou 74. L'ouverture de l'espace 78 est égal à l'épaisseur du pignon 18 que le dit espace 78 réceptionne.

Initialement, l'olivette 24 est monté dans son logement 33 situé dans le corps 26 du premier connecteur 12 et le verrou 22 est engagé dans la partie supérieure 31 taraudée de l'orifice 30 de manière non serré pour laisser l'olivette 24 libre de mouvement au sein de son logement 33. Le plot 60 est engagé sur l'extrémité filetée 62 de la tige 14 et le pignon 18 sur l'extrémité opposée au delà de l'orifice de préhension 64.

L'ensemble du dispositif est introduit dans le corps du patient par la voie d'abord pratiquée. Le premier connecteur 12 avec son olivette 24 et son verrou 22 est monté sur la tête 6 de la première des vis bicorticales 2 de manière à ce qu'elle soit complètement emprisonnée dans l'olivette 24, et que la face interne 38 de la dite olivette 24 soit en contact avec la face externe 10 de la tête 6 de la vis bicorticale 2 et la lèvre 44 en saillie en contact avec la collerette 8 de la vis 2. Le deuxième connecteur 16 est ensuite engagé complètement sur la tête 6 de la deuxième des vis bicorticales 2 par le trou 74. La face inférieure 80 du connecteur 16 est en contact avec la collerette 8 de la vis 2. La tige 14 est ensuite introduite, maintenue par son orifice de préhension 64, puis positionnée au fond des crochets bifides 28, 72 des premier 12 et deuxième 16 connecteurs respectivement de manière à ce que :
- le plot 60 s'insère dans l'espace 52 du premier connecteur 12, et avec l'une de ses faces 61 en contact avec la face d'appui 58,
- le pignon 18 s'insère dans l'espace 78 du deuxième connecteur 16, les dents débordant à l'intérieur de la chambre de réception 72 de la vis sans fin 20.

On serre alors le verrou 22. La face 36 de l'olivette 24 glisse alors sur la face inférieure 48 du verrou 22 forçant, grâce à la présence de la fente 40 et de la rainure 41, l'olivette 24 à se refermer donc à se resserrer sur la tête 6 de la vis bicorticale 2, bloquant l'ensemble du connecteur 12 en position sur la dite vis 2. La vis sans fin 20 est ensuite positionnée dans la chambre de réception 72 du second connecteur 16, les filets 21 venant en prise avec les dents du pignon 18. Le dispositif est alors prêt pour effectuer une distraction ou une contraction par mise en mouvement rotatif de la vis sans fin 20 au sein de la chambre de réception 72 du second connecteur 16.

Dans le cas de la distraction, la tige 14 entraînée en rotation par le pignon 18, lui-même manoeuvré par la vis sans fin 20, se dévisse dans le plot 60. Ce dernier vient en appui contre la face 52a du premier connecteur 12 alors que le pignon 18 vient en appui sur la face 78b du second connecteur 16. Les deux connecteurs sont obligés de s'éloigner l'un de l'autre, entraînant les vis bicorticales 2 et les vertèbres 100,200 où les vis 2 sont ancrées.

Dans le cas de la contraction, la tige 14 entraînée en rotation par le pignon 18 manoeuvré par la vis sans fin 20 se visse dans le plot 60. Ce dernier vient en appui contre la face 52b du premier connecteur alors que le pignon 18 vient en appui sur la face 78a du second connecteur 16. Les deux connecteurs sont obligés de se rapprocher l'un de l'autre, entraînant les vis bicorticales 2 et les vertèbres 100, 200 où les vis 2 sont ancrées.

Lors de ces mouvements, le pignon 18 ne peut ni s'extraire ni extraire la tige 14 du fait de l'angle α des branches 74, 75 du « U » du crochet de réception 72 du second connecteur 16 qui plaquent, via la tige 14, le pignon 18 contre la vis sans fin 20 sans possibilité d'échappement.

Une fois la distance désirée obtenue, on met en place entre les vertèbres un système d'ostéosynthèse rachidienne entre ces vertèbres comme, par exemple, des cages intersommatiques, puis on extrait du corps du patient par la voie d'abord pratiquée le dispositif objet de la présente invention. Enfin, l'arthrodèse est finalisée par la mise en place sur les organes d'ancrage d'un second système d'ostéosynthèse, comme, par exemple, un dispositif de fixation antérieure, qui assurera la stabilité du segment rachidien ainsi instrumenté durant la prise de la fusion au moyen du premier système d'ostéosynthèse.

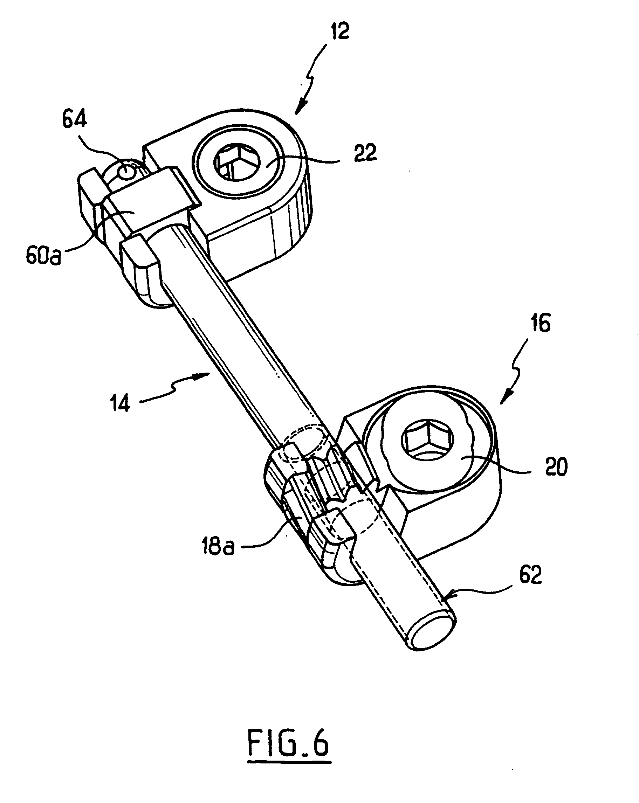

Le deuxième de réalisation est illustré par la figure 6. Il diffère du premier mode de réalisation précité dans le fait que :
- le plot 60a est solidaire de la tige 14 bloquant la dite tige 14 en rotation et en translation dans la direction de son propre axe par rapport au premier connecteur 12
- le pignon 18a se comporte comme un écrou dont le filetage est en prise avec celui 62 de la tige 14.

Les premier 12 et second 16 connecteurs sont identiques à ceux décrits dans le premier mode de réalisation. Le montage de ces connecteurs 12, 16 et de la tige 14 est similaire aussi. Lors de la distraction ou de la contraction, la tige 14 est immobile en rotation et c'est le pignon 18a qui en se vissant ou en se dévissant sur la tige 14 effectue les variations de distance entre les deux connecteurs 12, 16 du dispositif.

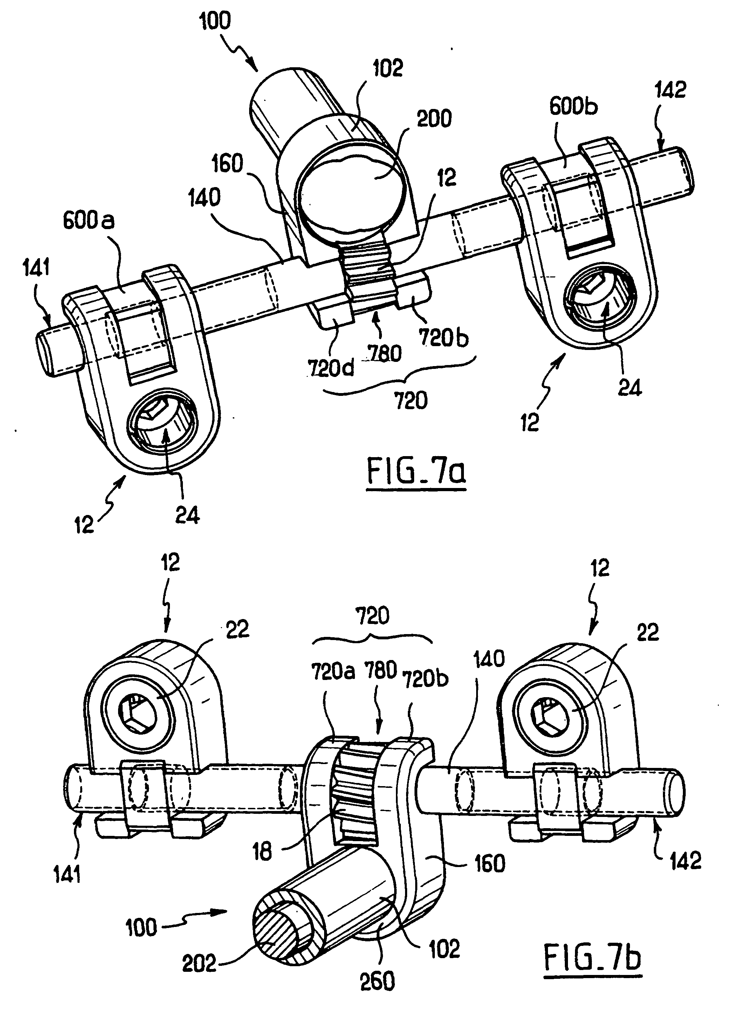

Le troisième mode de réalisation est illustré par les figures 7a et 7b. Les connecteurs placés sur les têtes 6 de vis bicorticales 2 sont identiques au premier connecteur 12 décrit dans le premier mode de réalisation précité. Le pignon 18 est solidaire de la tige 140 et est situé au milieu de la tige 140. Les extrémités de la tige 140 comportent chacune un filetage 141, 142. Les sens de ces filetages sont opposés l'un par rapport à l'autre. Un plot 600a, 600b comportant le filetage complémentaire est engagé sur chacun de ces filetages 141, 142. Le système comprend un connecteur 160 situé à l'extrémité d'un corps 102 d'instrument 100, similaire au second connecteur 16 décrit dans le premier mode de réalisation précité sauf en ce qui concerne la direction des branches du « U » du crochet de réception 720 qui sont parallèles à l'axe de la chambre de réception. Le corps 102 de l'instrument 100 est relié au connecteur 160 au niveau de son corps 260. Le système comprend une vis sans fin 200 solidaire d'un moyen d'entraînement 202 passant au travers du corps 102 de l'instrument 100.

Les connecteurs 12 sont mis en place comme précédemment. La tige 140 est ensuite installée dans les crochets de réception 28 des dits connecteurs 12 de manière à ce que les plots 600a, 600b soient dans l'espace 52 entre les crochets de réception 28 bifides respectifs comme décrit précédemment. L'instrument 100 est alors positionné sur la tige 140 au niveau du pignon 18, ce dernier se plaçant dans l'espace 780 du crochet de réception 720 bifide du connecteur 160 de l'instrument 100. Le pignon 18 se trouve en prise avec les filets de la vis sans fin 200.

Lors de la distraction, la tige 140, entraînée en rotation par le pignon 18, lui-même mu part la vis sans fin 20, se dévisse simultanément dans les deux plots 600a, 600b qui s'éloignent l'un de l'autre entraînant les connecteurs 12 en distraction.

Lors de la contraction, la tige 140, entraînée en rotation par le pignon 18 mu par la vis sans fin 20, se visse simultanément dans les deux plots 600a, 600b qui se rapprochent l'un de l'autre entraînant les connecteurs 12 en contraction.

Bien entendu, on pourra apporter à l'invention de nombreuse modifications sans sortir du cadre de celle-ci.

On pourra prévoir que :
- le plot est de section polygonale de manière générale ;
- le plot est de section circulaire ;
- le premier connecteur comprend un trou fileté de part en part en lieu et place du crochet bifide de réception et du plot ;
- la tige est solidaire du premier connecteur en lieu et place du crochet bifide et du plot ; ou encore que
- le deuxième connecteur a une liaison rotule avec son organe d'ancrage.

Les organes d'ancrage peuvent être des vis pédiculaires ou bien des crochets.

Dans chacun des exemples de réalisation qui précèdent, le système sera de préférence agencé pour être mécaniquement irréversible au niveau des engrènements entre les pièces. Ainsi, dans le premier mode, on actionne la vis sans fin 20 pour déplacer les organes d'ancrage aux vertèbres. Mais aucune sollicitation sur ces dernières ne peut en retour mouvoir les pièces, notamment la vis sans fin, en sens inverse. Il en est de même dans le deuxième mode. Dans le troisième mode, le moyen d'entraînement 202 permet de déplacer les deux organes d'ancrage aux vertèbres, mais pas le contraire. Les conditions pour l'obtention d'une telle irréversibilité sont connues en soi dans d'autres domaines. L'irréversibilité dépend notamment de l'inclinaison des dents et des filets des pièces en engrènement.

## Revendications

1. Dispositif de distraction et/ou de contraction chirurgical rachidien, comportant des éléments, des moyens de jonction entre ces éléments constitués par une tige filetée et des moyens de réglages d'une distance entre ces éléments **caractérisé en ce que** les moyens de réglage comprennent un assemblage à pignon (18;18a) et vis sans fin (20;200), et les éléments sont des connecteurs (12,16) aptes à être fixés à des organes (2) d'ancrage vertébral respectifs.

2. Dispositif selon la revendication 1 **caractérisé en ce que** les moyens de réglage d'une distance comprennent une tige (14;140) apte à relier les connecteurs (12,16).

3. Dispositif selon la revendication 2 **caractérisé en ce que** la tige (14;140) est libre en translation et rotation conjuguées selon son axe propre par rapport à l'un des connecteurs (12,16).

4. Dispositif selon la revendication 3 **caractérisé en ce que** la tige (14) est libre en rotation selon son axe propre par rapport à l'autre des connecteurs (12,16).

5. Dispositif selon la revendication 3 **caractérisé en ce que** la tige (140) est libre en translation et rotation conjuguées selon son propre axe par rapport à l'autre des connecteurs (12,16) avec une hélice de sens opposée.

6. Dispositif selon la revendication 2 **caractérisé en ce que** la tige (14) est bloquée en translation et en rotation suivant son axe propre par rapport à l'un des connecteurs (12,16).

7. Dispositif selon la revendication 6 **caractérisé en ce que** la tige (14) est libre en translation selon son axe propre par rapport à l'autre des connecteurs (12,16).

8. Dispositif selon la revendication 3, 4 ou 5
**caractérisé en ce que** le pignon (18) est rigidement solidaire de la tige (14;140).

9. Dispositif selon la revendication 6 ou 7
**caractérisé en ce que** le pignon (18a) forme une liaison vis/écrou avec la tige (14).

10. Dispositif selon l'une des revendications 2 à 9 **caractérisé en ce qu'**il comporte des moyens de liaison entre la tige (14) et les connecteurs (12,16), ces moyens de liaison comprenant le pignon (18;18a).

11. Dispositif selon l'une des revendications 2 à 10 **caractérisé en ce qu'**il comporte des moyens de liaison entre la tige (14;140) et les connecteurs (12,16), ces moyens de liaison comprenant un plot (60;600a,600b) qui forme butée pour le mouvement de translation et/ou de rotation de la tige par rapport au connecteur selon l'axe de la tige.

12. Dispositif selon la revendication 11 **caractérisé en ce que** le plot présente une section polygonale, de préférence à quatre cotés (61).

13. Dispositif selon l'une des revendications 10 à 12 **caractérisé en ce que** l'un au moins des connecteurs (12,16) présente un crochet (28,72) bifide délimitant un espace (52,78) apte à recevoir la tige (14;140) et ses moyens de liaison (18;18a;60;600a,600b) avec le connecteur.

14. Dispositif selon les revendications 12 et 13 **caractérisé en ce qu'**il est agencé de sorte que, lorsque le plot (60;600a,600b) est reçu, l'un des cotés (61) de la section polygonale de la butée (60;600a,600b) est apte à venir en contact avec le fond (58) de l'espace (52) du crochet bifide (28).

15. Dispositif selon l'une des revendications 2 à 14 **caractérisé en ce que** l'un des connecteurs (12,16) comprend une bague (24) à face sphérique.

16. Dispositif selon la revendication 15 **caractérisé en ce que** le connecteur comprend une chambre (33) apte à recevoir à rotation la bague (24).

17. Dispositif selon la revendication 15 ou 16 **caractérisé en ce que** le connecteur comprend un verrou (22) apte à bloquer en rotation la bague (24).

18. Dispositif selon la revendication 17 **caractérisé en ce que** le verrou (22) comprend une face sphérique (48) concave apte à venir en contact avec la face sphérique de la bague (24).

19. Dispositif selon l'une des revendications 2 à 18 **caractérisé en ce que** l'un des connecteurs (12,16) est adjacent à l'assemblage à pignon et vis sans fin.

20. Dispositif selon la revendication 19 **caractérisé en ce que** la vis sans fin (20) est apte à être reçue à rotation dans le connecteur.

21. Dispositif selon l'une des revendications 1 à 18 **caractérisé en ce qu'**il comporte en outre un instrument (100), en vue de la manoeuvre de la vis sans fin, apte à recevoir à rotation la vis sans fin (200).

22. Dispositif selon la revendication 21 **caractérisé en ce que** l'instrument (100) comprend un crochet (720) bifide, délimitant un espace (780), apte à recevoir la tige (14;140) et le pignon (18) rigidement solidaire de la tige.

23. Système d'ostéosynthèse rachidienne comprenant deux organes d'ancrage vertébral, **caractérisé en ce qu'**il comprend un dispositif de distraction et/ou de contraction selon l'une quelconque des revendications précédentes.

24. Système selon la revendication 23 **caractérisé en ce que** l'un des connecteurs (12,16) est apte à former une liaison rotule avec l'organe d'ancrage vertébral (2) associé.

25. Système selon la revendication 24 **caractérisé en ce que** la bague (24) est apte à être montée sur l'organe d'ancrage vertébral (2).

## Patentansprüche

1. Vorrichtung zur chirurgischen Distraktion und/oder Kontraktion am Rückgrat, die Elemente, eine Verbindung zwischen diesen Elementen herstellende Mittel, die durch eine mit einem Gewinde versehene Stange gebildet werden und eine Mittel zum Einstellen eines Abstands zwischen diesen Elementen umfaßt **dadurch gekennzeichnet, daß** die Einstellmittel eine Anordnung aus einem Zahnritzel (18; 18a) und einer Endlosschraube (20; 200) umfassen und die Elemente Verbindungsstücke (12, 16) sind, die so ausgestaltet sind, daß sie an jeweils zugehörigen Organen (2) zur Wirbelverankerung befestigt werden können.

2. Vorrichtung nach Anspruch 1, **dadurch gekennzeichnet, daß** die Mittel zum Einstellen eines Abstands eine Stange (14; 140) umfassen, die dazu geeignet ist die Verbindungsstücke (12, 16) zu verbinden.

3. Vorrichtung nach Anspruch 2, **dadurch gekennzeichnet, daß** die Stange (14; 140) bezüglich eines der Verbindungsstücke (12, 16) frei für kombinierte Verschiebungen und Drehungen entlang ihrer und um ihre eigenen Achse ist.

4. Vorrichtung nach Anspruch 3, **dadurch gekennzeichnet, daß** die Stange (14) bezüglich des anderen Verbindungsstücks (12, 16) frei für Drehungen um ihre eigene Achse ist.

5. Vorrichtung nach Anspruch 3, **dadurch gekennzeichnet, daß** die Stange (140) bezüglich des anderen Verbindungsstücks (12, 16) frei für kombinierte Verschiebungen und Drehungen entlang ihrer und um ihre eigenen Achse mit einem Schraubengang in umgekehrter Richtung ist.

6. Vorrichtung nach Anspruch 2, **dadurch gekennzeichnet, daß** die Stange (14) bezüglich eines der Verbindungsstücke (12, 16) für Verschiebungen und für Drehungen entlang ihrer und um ihre eigenen Achse festgestellt ist.

7. Vorrichtung nach Anspruch 6, **dadurch gekennzeichnet, daß** die Stange (14) bezüglich des anderen Verbindungsstücks (12, 16) frei für Verschiebungen entlang ihrer eigenen Achse ist.

8. Vorrichtung nach Anspruch 3, 4 oder 5, **dadurch gekennzeichnet, daß** das Zahnritzel (18) mit der Stange (14; 140) starr verbunden ist.

9. Vorrichtung nach Anspruch 6 oder 7, **dadurch gekennzeichnet, daß** das Zahnritzel (18a) mit der Stange (14) eine Schraube/Mutter-Verbindung bildet.

10. Vorrichtung nach einem der Ansprüche 2 bis 9, **dadurch gekennzeichnet, daß** sie Mittel zur Verbindung zwischen der Stange (14) und den Verbindungsstücken (12, 16) umfaßt, wobei diese Verbindungsmittel das Zahnritzel (18, 18a) umfassen.

11. Vorrichtung nach einem der Ansprüche 2 bis 10, **dadurch gekennzeichnet, daß** sie Mittel zur Verbindung zwischen der Stange (14; 140) und den Verbindungsstücken (12, 16) umfaßt, wobei diese Verbindungsmittel ein Kontaktstück (60; 600a, 600b) umfassen, das eine Abstützung für die Verschiebungs- und/oder Drehbewegung der Stange in Bezug auf das Verbindungsstück gemäß der Achse der Stange bildet.

12. Vorrichtung nach Anspruch 11, **dadurch gekennzeichnet, daß** das Kontaktstück eine polygonale Schnittfläche mit vorzugsweise vier Seiten (61) aufweist.

13. Vorrichtung nach einem der Ansprüche 10 bis 12, **dadurch gekennzeichnet, daß** wenigstens eines der Verbindungsstücke (12, 16) einen zweiarmig Haken (28, 72) aufweist, der einen Raum (52, 78) abgrenzt, der geeignet ist, die Stange (14; 140) und ihre Mittel zur Verbindung (18; 18a; 60; 600a, 600b) mit dem Verbindungsstück aufzunehmen.

14. Vorrichtung nach den Ansprüchen 12 und 13, **dadurch gekennzeichnet, daß** sie derart ausgestaltet ist, daß wenn das Kontaktstück (60; 600a, 600b) aufgenommen wird, eine der Seiten (61) der polygonalen Schnittfläche der Abstützung (60; 600a, 600b) dazu geeignet ist, mit dem Boden (58) des Raums (52) des zweiarmig Hakens (28) in Kontakt zu kommen.

15. Vorrichtung nach einem der Ansprüche 2 bis 14, **dadurch gekennzeichnet, daß** eines der Verbindungsstücke (12, 16) eine Hülse (24) mit einer sphärischen Fläche umfaßt.

16. Vorrichtung nach Anspruch 15, **dadurch gekennzeichnet, daß** das Verbindungsstück eine Kammer (33) umfaßt, die dazu ausgestaltet ist, die Hülse (24) drehbar aufzunehmen.

17. Vorrichtung nach Anspruch 15 oder 16, **dadurch gekennzeichnet, daß** das Verbindungsstück eine Verriegelung (22) umfaßt, die dazu ausgestaltet ist, Drehungen der Hülse (24) zu blockieren.

18. Vorrichtung nach Anspruch 17, **dadurch gekennzeichnet, daß** die Verriegelung (22) eine konkave kugelförmige Fläche (48) umfaßt, die dazu ausgestaltet ist, mit der kugelförmigen Fläche der Hülse (24) in Kontakt zu kommen.

19. Vorrichtung nach einem der Ansprüche 2 bis 18, **dadurch gekennzeichnet, daß** eines der Verbindungsstücke (12, 16) benachbart zur Anordnung aus dem Zahnritzel und der Endlosschraube ist.

20. Vorrichtung nach Anspruch 19, **dadurch gekennzeichnet, daß** die Endlosschraube (20) dazu ausgestaltet ist, drehbar im Verbindungsstück aufgenommen zu werden.

21. Vorrichtung nach einem der Ansprüche 1 bis 18, **dadurch gekennzeichnet, daß** sie außerdem ein Instrument (100) umfaßt, um die Endlosschraube zu handhaben, das dazu ausgestaltet ist, die Endlosschraube (200) drehbar aufzunehmen.

22. Vorrichtung nach Anspruch 21, **dadurch gekennzeichnet, daß** das Instrument (100) einen zweiarmig Haken (720) umfaßt, der einen Raum (780) abgrenzt, der dazu ausgestaltet ist, die Stange (14; 140) und das starr mit der Stange verbundene Zahnritzel (18) aufzunehmen.

23. Osteosynthesesystem für das Rückgrat, das zwei Organe zur Wirbelverankerung umfaßt, **dadurch gekennzeichnet, daß** es eine Vorrichtung zur Distraktion und/oder Kontraktion nach einem der vorhergehenden Ansprüche umfaßt.

24. System nach Anspruch 23, **dadurch gekennzeichnet, daß** eines der Verbindungsstücke (12, 16) dazu ausgestaltet ist, mit dem zugehörigen Organ zur Wirbelverankerung (2) eine Kugelgelenkverbindung auszubilden.

25. System nach Anspruch 24, **dadurch gekennzeichnet, daß** die Hülse (24) dazu ausgestaltet ist, auf dem Organ zur Wirbelverankerung (2) angebracht zu werden.

## Claims

1. Spinal surgery distraction and/or contraction device, comprising elements, means for linking these elements being formed by a threaded rod and means for adjusting a distance between these elements, **characterized in that** the adjustment means comprise an assembly of pinion (18; 18a) and endless screw (20; 200), and the elements are connectors (12, 16) designed to be fixed to respective vertebral anchoring members (2).

2. Device according to Claim 1, **characterized in that** the means for adjustment of a distance comprise a rod (14; 140) designed to link the connectors (12, 16).

3. Device according to Claim 2, **characterized in that** the rod (14; 140) is free both in translation and rotation on its own axis relative to one of the connectors (12, 16).

4. Device according to Claim 3, **characterized in that** the rod (14) is free in rotation about its own axis relative to the other of the connectors (12, 16).

5. Device according to Claim 3, **characterized in that** the rod (140) is free both in translation and rotation on its own axis relative to the other of the connectors (12, 16) with a helix of opposite direction.

6. Device according to Claim 2, **characterized in that** the rod (14) is immobilized in translation and in rotation on its own axis relative to one of the connectors (12, 16).

7. Device according to Claim 6, **characterized in that** the rod (14) is free in translation on its own axis relative to the other of the connectors (12, 16).

8. Device according to Claim 3, 4 or 5, **characterized in that** the pinion (18) is rigidly integral with the rod (14; 140).

9. Device according to Claim 6 or 7, **characterized in that** the pinion (18a) forms a screw/nut link with the rod (14).

10. Device according to one of Claims 2 to 9, **characterized in that** it comprises linking means between the rod (14) and the connectors (12, 16), these linking means comprising the pinion (18; 18a).

11. Device according to one of Claims 2 to 10, **characterized in that** it comprises linking means between the rod (14; 140) and the connectors (12, 16), these linking means comprising a block (60; 600a, 600b) which forms an abutment for the translation and/or rotation movement of the rod relative to the connector on the axis of the rod.

12. Device according to Claim 11, **characterized in that** the block has a polygonal cross section, preferably with four sides (61).

13. Device according to one of Claims 10 to 12, **characterized in that** at least one of the connectors (12, 16) has a bifid hook (28, 72) delimiting a space (52, 78) designed to receive the rod (14; 140) and its means of linkage (18; 18a; 60; 600a, 600b) to the connector.

14. Device according to Claims 12 and 13, **characterized in that** it is designed in such a way that when the block (60; 600a, 600b) is received, one of the sides (61) of the polygonal cross section of the abutment (60; 600a, 600b) is able to come into contact with the bottom (58) of the space (52) of the bifid hook (28).

15. Device according to one of Claims 2 to 14, **characterized in that** one of the connectors (12, 16) comprises a ring (24) with a spherical face.

16. Device according to Claim 15, **characterized in that** the connector comprises a chamber (33) designed to receive the ring (24) with rotation.

17. Device according to Claim 15 or 16, **characterized in that** the connector comprises a locking means (22) designed to immobilize the ring (24) in rotation.

18. Device according to Claim 17, **characterized in that** the locking means (22) comprises a concave spherical face (48) designed to come into contact with the spherical face of the ring (24).

19. Device according to one of Claims 2 to 18, **characterized in that** one of the connectors (12, 16) is adjacent to the pinion and endless screw assembly.

20. Device according to Claim 19, **characterized in that** the endless screw (20) is designed to be received with rotation in the connector.

21. Device according to one of Claims 1 to 18, **characterized in that** it additionally comprises an instrument (100), for manoeuvring the endless screw, and designed to receive the endless screw (200) with rotation.

22. Device according to Claim 21, **characterized in that** the instrument (100) comprises a bifid hook (720) delimiting a space (780), designed to receive the rod (14; 140) and the pinion (18) rigidly integral with the rod.

23. Spinal osteosynthesis system comprising two vertebral anchoring members, **characterized in that** it comprises a distraction and/or contraction device according to any one of the preceding claims.

24. System according to Claim 23, **characterized in that** one of the connectors (12, 16) is designed to form a ball and socket link with the associated vertebral anchoring member (2).

25. System according to Claim 24, **characterized in that** the ring (24) is designed to be fitted on the vertebral anchoring member (2).
